(19) Europäisches Patentamt
European Patent Office
Office européen des brevets

(11) Veröffentlichungsnummer: **0 000 005**
**B1**

(12) # EUROPÄISCHE PATENTSCHRIFT

(21) Anmeldenummer: **78100078.1**

(22) Anmeldetag: **01.06.78**

(51) Int. Cl.³: **C 07 D 501/36**
A 61 K 31/545

(54) **Neue Cephalosporinderivate, Verfahren zu ihrer Herstellung und pharmazeutische Präparate**

(30) Priorität: **03.06.77 LU 77485**
**22.03.78 CH 3142/78**

(43) Veröffentlichungstag der Anmeldung:
**20.12.78 Patentblatt 78/01**

(45) Bekanntmachung des Hinweises auf die Patenterteilung:
**29.10.80 Patentblatt 80/22**

(84) Benannte Vertragsstaaten:
**BE CH DE FR LU NL SE**

(56) Entgegenhaltungen:
**FR - A - 2 137 899**
**FR - A - 2 275 215**
**FR - A - 2 280 381**

(73) Patentinhaber: **F. HOFFMANN-LA ROCHE & CO. Aktiengesellschaft**
**CH - 4002 Basel (CH)**

(72) Erfinder: **Montavon, Marc, Dr.**
**Realpstrasse 72**
**CH - 4054 Basel (CH)**
**Reiner, Roland, Dr.**
**Rheinfelderstrasse 8**
**CH - 4058 Basel (CH)**

(74) Vertreter: **Lederer, Franz, Dr. et al**
**Patentanwälte Lederer,**
**Meyer Lucile-Grahn-Strasse 22**
**D - 8000 München 80 (DE)**

**EP 0 000 005 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Cephalosporinderivate, Verfahren zu ihrer Herstellung und pharmazeutische Präparate

Die vorliegende Erfindung betrifft neue Acylderivate der allgemeinen Formel

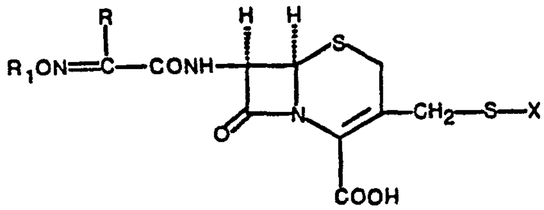

I

in der R Furyl, Thienyl oder ggf. durch Halogen, Hydroxy, niederes Alkoxy oder niederes Alkyl substituiertes Phenyl, $R_1$ niederes Alkyl oder Aminocarbonylmethyl und X eine Gruppe der Formeln

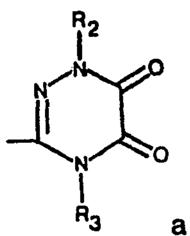

a

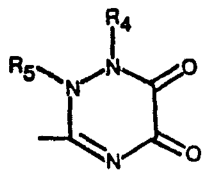

b

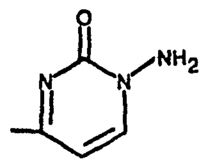

c

in denen einer der beiden Reste $R_2$ und $R_3$ bzw. $R_4$ und $R_5$ Wasserstoff und der andere niederes Alkyl, Carboxymethyl oder Sulfomethyl darstellt, bedeutet,
sowie Salze dieser Verbindungen und Hydrate dieser Salze.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthyl-äthylendiamin, Alkylaminen oder Dialkylaminen, sowie Salze mit Aminosäuren, wie z.B. Salze mit Arginin oder Lysin. Die Salze können Monosalze oder auch Disalze sein. Die zweite Salzbildung erfolgt an der tautomeren Enolform des Triazinrestes b, welche sauren Charakter hat.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Minderalsäuresalze, wie Sulfate, Nitrate, Phosphate und dgl., Alkyl- und Mono-arylsulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Abscorbate und dgl.

Die Salze der Verbindungen der Formel I können hydratisiert sein. Die Hydratisierung kann im Zuge des Herstellungsverfahrens erfolgen oder allmählich als Folge hygroskopischer Eigenschaften eines zunächst wasserfreien Salzes einer Verbindung der Formel I auftreten.

Die vorstehend genannten niederen Alkylgruppen sind entweder geradkettig oder verzweigt und können bis zu 7 Kohlenstoffatome enthalten, z.B. Methyl, Aethyl, n-Propyl, Isopropyl, n-Pentyl, n-Heptyl. Niedere Alkoxygruppen haben analoge Bedeutung. Halogen stellt alle vier Halogene dar, d.h. Fluor, Chlor, Brom und Jod; bevorzugt sind Chlor und Brom.

Bevorzugte R-Gruppen sind Furyl, Thienyl und Phenyl, insbesondere Furyl. Als $R_1$ ist Methyl bevorzugt. Bevorzugte X-Gruppen sind die Gruppe (c) sowie die Gruppen (a) und (b), worin einer der beiden Reste $R_2$ und $R_3$ bzw. $R_4$ und $R_5$ Wasserstoff und der andere Methyl darstellt. Besonders bevorzugte X-Gruppen sind die 1,2,5,6-Tetrahydro-2-methyl-5,6-dioxo-as-triazin-3-yl- und die 1,4,5,6 - Tetrahydro - 4 - methyl - 5,6 - dioxo - as - triazin - 3 - yl - gruppe.

Bevorzugte Verbindungen sind (7R) - 7 - [2 - (2 - Furyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure und deren Salze sowie die Hydrate dieser Salze.

Die Verbindungen der Formel I sowie deren Salze bzw. Hydrate der Salze können in der synisomeren Form

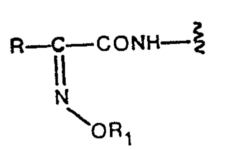

oder in der anti-isomeren Form

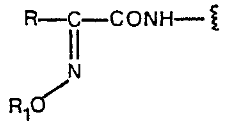

bzw. als Gemische dieser beiden Formen vorliegen. Bevorzugt ist die syn-isomere Form bzw. Gemische, in denen die syn-isomere Form überwiegt.

Die Acylderivate der Formel I sowie deren Salze bzw. Hydrate dieser Salze werden erfindungsgemäss durch ein Verfahren hergestellt, welches dadurch gekennzeichnet ist, dass man

a) eine Verbindung der allgemeinen Formel

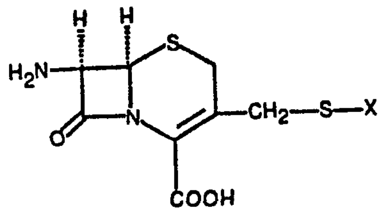

II

in der X die in Formel I gegebene Bedeutung hat und die Carboxygruppe in geschützter Form vorliegen kann, mit einer Säure der allgemeinen Formel

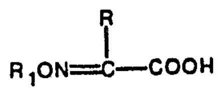

III

in der R und $R_1$ die in Formel I gegebene Bedeutung haben, oder mit einem reaktionsfähigen funktionellen Derivat dieser Säure umsetzt und die Schutzgruppe abspaltet, oder dass man

b) eine Verbindung der allgemeinen Formel

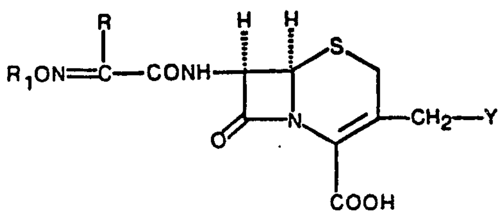

IV

in der R und $R_1$ die in Formel I gegebene Bedeutung haben, Y eine austretende Gruppe darstellt und die Carboxygruppe in geschützter Form vorliegen kann, mit einem Thiol der allgemeinen Formel

HS—X V

in der X die in Formel I gegebene Bedeutung hat, umsetzt und die Schutzgruppe abspaltet, wonach man das Reaktionsprodukt ggfs. in ein Salz bzw. ein Hydrat dieses Salzes überführt.

Die in den Ausgangsverbindungen der Formeln II and IV vorhandenen Carboxygruppen können wahlweise geschützt sein, z.B. durch Veresterung zu einem leicht spaltbaren Ester, wie einem Silylester, z.B. dem Trimethylsilylester. Die Carboxygruppe kann auch durch Salzbildung mit einer anorganischen oder tertiären organischen Base, wie Triäthylamin, geschützt werden.

Als reaktionsfähige funktionelle Derivate von Säuren der Formel III kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxy-succinimidester, und Amide, z.B. Imidazolide, in Betracht.

Als austretende Gruppe Y einer Verbindung der Formel IV kommen beispielsweise Halogene, z.B. Chlor, Brom oder Jod, Acyloxyreste, z.B. niedere Alkanoylreste, wie Acetoxy, niedere Alkyl- oder Arylsulfonyloxyreste, wie Mesyloxy oder Tosyloxy, oder der Azidorest in Frage.

Die Umsetzung einer Verbindung der Formel II mit einer Säure der Formel III oder einem reaktionsfähigen funktionellen Derivat davon kann in an sich bekannter Weise durchgeführt werden. So kann man z.B. eine freie Saüre der Formel III mit einem der erwähnten Ester entsprechend Formel II mittels eines Carbodiimids, wie Dicyclohexylcarbodiimid, in einem inerten Lösungsmittel, wie Essigester, Acetonitril, Dioxan, Chloroform, Methylenchlorid, Benzol oder Dimethylformamid kondensieren und anschliessend die Estergruppe abspalten. Anstelle von Carbodiimiden lassen sich als

Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel II, z.B. ein Trialkylammoniumsalz, mit einem reaktionsfähigen funktionellen Derivat einer Säure der Formel III wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel III mit dem Amin der Formel II umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet; es kann aber auch in einem aprotischen organischen Lösungsmittel, wie z.B. Dimethylformamid, Dimethylsulfoxid, oder Hexamethylphosphorsäuretriamid, gearbeitet werden.

Die Umsetzung einer Verbindung der Formel II mit einer Verbindung der Formel III oder einem reaktionsfähigen funktionellen Derivat davon kann zweckmässig bei Temperaturen zwischen etwa −40°C und Zimmertemperatur, beispielsweise bei etwa 0–10°C, erfolgen.

Die Umsetzung einer Verbindung der Formel IV mit einem Thiol der Formel V kann in an sich bekannter Weise, z.B. bei einer Temperatur zwischen etwa 40 und 80°C, zweckmässig bei etwa 60°C, in einem polaren Lösungsmittel, beispielsweise in einem Alkohol, wie z.B. in einem niederen Alkanol, wie Aethanol, Propanol und dgl., in Dimethylformamid oder Dimethylsulfoxid, vorzugsweise in Wasser oder in einer Pufferlösung mit einem pH von etwa 6 bis 7, vorzugsweise 6,5, durchgeführt werden.

Nach erfolgter Umsetzung einer Verbindung der Formel II oder IV mit einer Verbindung der Formel II bzw. V wird die allenfalls vorhandene Schutzgruppe abgespalten.

Wenn die Schutzgruppe eine Silylgruppe darstellt (Silylester), kann diese Gruppe besonders leicht durch Behandlung des Umsetzungsproduktes mit Wasser abgespalten werden. Wenn die Carboxylgruppe einer Säure der Formel IV durch Salzbildung (z.B. mit Triäthylamin) geschützt ist, so kann die Abspaltung dieser salzbildenen Schutzgruppe durch Behandlung mit Säure erfolgen. Als Säure kann hierbei z.B. Salzsäure, Schwefelsäure, Phosphorsäure oder Citronensäure verwendet werden.

Die als Ausgangsprodukte verwendeten 7-Aminoverbindungen der Formel II können ausgehend von einer Verbindung der Formel

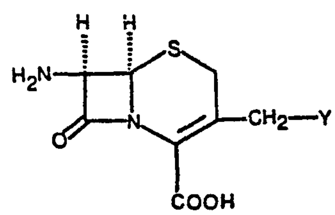

VI

in der Y eine austretende Gruppe darstellt und die Carboxygruppe in geschützter Form vorliegen kann, mit einem Thiol der Formel V hergestellt werden. Die Umsetzung kann unter den gleichen Bedingungen wie diejenige der Ausgangsverbindungen IV und V erfolgen.

Ein allenfalls erhaltenes syn/anti-Gemisch einer Verbindung der Formel I kann in die entsprechenden syn- und anti-Formen in üblicher Weise getrennt werden, beispielsweise durch Umkristallisation oder durch chromatographische Methoden unter Verwendung eines geeigneten Lösungsmittels bzw. Lösungsmittelgemisches.

Aus der französischen Patentpublikation No. 2.137.899 sind Cephalosporinderivate bekannt, welche in 7-Stellung u.a. einen Substituenten des vorliegenden Typs $R_1ON{=}CR{-}CONH{-}$ tragen können. Andererseits sind aus den französischen Patentpublikationen Nos. 2.275.215 und 2.280.381 Cephalosporinderivate bekannt, welche in 3-Stellung einen substituierten Thiomethylrest des vorliegenden Typs $—CH_2—S—X$ tragen können. Es wurde nun gefunden, daß die neuen Cephalosporinderivate der Formel I sowie deren Salze und die Hydrate dieser Salze, in welchen erstmals die erwähnten Substituenten in den Stellungen 7 und 3 gleichzeitig vorliegen, wertvolle antibiotische, insbesondere bakterizide Wirksamkeit besitzen. Sie besitzen ein breites Wirkungsspektrum gegen Grampositive und Gram-negative Mikroorganismen, einschließlich ß-Lactamase bildende Staphylokokken und verschiedene ß-Lactamase bildende Gram-negative Bakterien, wie z.B. Haemophilus influenzae, Escherichia coli, Proteus- und Klebsiella-Spezies.

Die Verbindungender Formel I sowie die pharmazeutisch verträglichen Salze und hydratisierten Formen davon können zur Behandlung und Prophylaxe von Infektionskrankheiten verwendet werden. Für den Erwachsenen kommt eine Tagesdosis von etwa 1 g bis etwa 4 g in Betracht. Die parenterale Verabreichung der erfindungsgemäßen Verbindungen ist besonders bevorzugt.

Zum Nachweis der antimikrobiellen Wirksamkeit der erfindungsgemässen Verbindungen wurden folgende repräsentative Vertreter getestet:

Produkt A: (7R) - 7 - [2 - (2 - Furyl) - 2 - (methoxyimino) - acetamido] - 3 -/[(1,2,5,6 - tetrahydro - 2 - methyl - 5,6 - dioxo - as - triazin - 3 - yl)thio]methyl/ - 3 - cephem - 4-carbonsäure

Produkt B: (7R) - 7 - [2 - (2 - furyl) - 2 - (methoxyimino)acetamido] - 3 -/[1 - amino - 1,2 - dihydro - 2 - oxo - 4 - pyrimidinyl) - thio] - methyl/ - 3 - cephem - 4 - carbonsäure

Produkt C: (7R) - 7 - [2 - (Phenyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4-carbonsäure

Produkt D: (7R) - 7 - [2 - (2 - Furyl) - 2 - (methoxyimino)acetamido] - 3 - /[(1,4,5,6 - tetrahydro - 4 - methyl - 5,6 - dioxo - as - triazin - 3 - yl)thio]methyl/ - 3 - cephem - 4-carbonsäure

Produkt E: (7R) - 7 - [2 - (Methoxyimino) - 2 - (2 - thienyl)acetamido] - 3 - /[(1,2,5,6, - tetrahydro - 2 - methyl - 5,6 - dioxo - as - triazin - 3 - yl)thio]methyl/ - 3 - cephem - 4-carbonsäure

Produkt F: (7R) - 3 - /[(1 - Aethyl - 1,4,5,6 - tetrahydro - 5,6 - dioxo - as - triazin - 3 - yl)thio] methyl/ - 7 - [2 - (2 - furyl) - 2 - (methoxyimino)acetamido] - 3 - cephem - 4-carbonsäure

Produkt G: (7R) - 7 - /2 - [(Carbamoylmethoxy)imino] - 2 - (2 - furyl) - acetamido/ - 3 - /[(1, 4,5,6 - tetrahydro - 4 - methyl - 5,6 - dioxo - as - triazin - 3 - yl)thio]methyl/ - 3-cephem - 4 - carbonsäure.

Aktivität in vitro: Mindesthemmkonzentration ($\mu g/ml$)

| | | A | B | C | D | E | F | G |
|---|---|---|---|---|---|---|---|---|
| Haemophilus influenzae | Stamm 1 | 1.2 | 2.5 | 2.5 | 2.5 | 2.5 | 2.5 | 5.0 |
| | Stamm 2 | 0.16 | 0.63 | 0.16 | 0.31 | 0.16 | 0.31 | 0.63 |
| | Stamm 3 | 0.16 | 0.63 | 0.16 | 0.31 | 0.16 | 1.2 | 0.31 |
| | Stamm 4 | 0.16 | 0.31 | 0.16 | 0.31 | 0.16 | 0.16 | 0.63 |
| | Stamm 5 | 0.08 | 0.31 | 0.08 | 0.16 | 0.08 | 0.08 | 0.31 |
| | Stamm 6 | 0.16 | 0.31 | 0.08 | 0.16 | 0.08 | 0.08 | 0.63 |
| | Stamm 7 | 0.08 | 0.31 | 0.08 | 0.16 | 0.08 | 0.16 | 0.63 |
| Klebsiella pneumoniae | | 10 | 20 | 20 | 5 | 40 | 40 | 2.5 |
| Escherichia coli | Stamm 1 | 0.63 | 2.5 | 1.2 | 0.16 | 2.5 | 1.2 | 0.31 |
| | Stamm 2 | 40 | 20 | 10 | 5 | 20 | 80 | 5 |
| Proteus mirabilis | Stamm 1 | 2.5 | 10 | 10 | 10 | 10 | 20 | 5 |
| | Stamm 2 | 5 | 40 | 20 | 20 | 20 | 40 | 10 |
| Proteus vulgaris | | 5 | 40 | 10 | 1.2 | 10 | 20 | 1.2 |
| Proteus rettgeri | | 0.63 | 2.5 | 0.63 | 0.63 | 2.5 | 10 | 0.63 |
| Staphylococcus aureus ATCC 6538 | | 2.5 | 0.16 | 2.5 | 0.63 | 2.5 | 5 | 1.2 |
| penicillinresistenter Stamm | | 5 | 1.2 | 2.5 | 0.63 | 2.5 | 5 | 1.2 |

Aktivität in vivo

Gruppen von 10 Mäusen werden mit einer wässrigen Suspension von Proteus mirabilis intraperitoneal infiziert. Eine Stunde nach der Infektion wird die Prüfsubstanz subcutan appliziert. Die Zahl der überlebenden Tiere wird am 4. Tag bestimmt. Es werden verschiedene Dosierungen appliziert, und durch Interpolation wird diejenige Dosis bestimmt, bei der 50% der Versuchstiere überlebten ($CD_{50}$, mg/kg).

| Prüfsubstanz | A | B | C | D | E | F | G |
|---|---|---|---|---|---|---|---|
| $CD_{50}$, mg/kg | 0,09 | 3,0 | 0,60 | 0,70 | 1,15 | 0,85 | 0,80 |

Toxizität (Maus, 24/Stundenwerte)

| Prüfsubstanz | A | B | C | D | E | F | G |
|---|---|---|---|---|---|---|---|
| $LD_{50}$, mg/kg | | | | | | | |
| i.v. | 500–1000 | 1000–2000 | 1000–2000 | 500–1000 | 2000–4000 | 1000–2000 | >4000 |
| s.c. | >4000 | | | >4000 | | | |
| p.o. | >5000 | | | >5000 | | | |

Pharmazeutische Präparate, vorzugsweise Trockenampullen, können die Verbindungen der Formel I, ihre Salze oder hydratisierte Formen dieser Salze, eventuell in Mischung mit einem anderen therapeutisch wertvollen Stoff enthalten. Zweckmässig sind sie mit einem insbesondere für die parenterale Applikation geeigneten pharmazeutischen, anorganischen oder organischen inerten Trägermaterial vermischt, wie z.B. mit Wasser, Gummi arabicum. Die pharmazeutischen Präparate leigen vorzugsweise in flüssiger Form vor, z.B. als Lösungen, Suspensionen oder Emulsionen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer.

Beispiel 1

Herstellung des Natriumsalzes der (7R) - 7 - [2 - (2 - Furyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure:

5,60 g 2-Methoxyimino-2-furyl-essigsäure (syn/anti-Gemisch 80:20) werden in 150 ml Benzol gelöst und unter Stickstoff-Begasung bei 5—10°C mit 4,2 ml Triäthylamin, 2,6 ml Oxalylchlorid und 6 Tropfen Dimethylformamid versetzt. Das Gemisch wird 1 Stunde bei 5—10°C und 1/2 Stunde bei 25°C unter Stickstoff-Begasung gerührt und danach im Vakuum bei 40°C eingedampft. Der Rückstand wird in 150 ml Aceton suspendiert und bei 0°C mit einer Lösung von 11,2 g (7R) - 7 - Amino - 3 - desacetoxy - 3 - [(1,2,5,6 - tetrahydro - 2 - methyl - 5,6 - dioxo - as - triazin - 3 - yl) - thio] - cephalosporansäure in 150 ml Wasser, welche zuvor mittels 2-n wässriger Natronlauge auf ph 7,5 eingestellt worden war, versetzt. Das Reaktionsgemisch wird 2 1/2 Stunden unter Stickstoff und bei 0—10°C gerührt, wobei das pH mittels Zugabe von 2-n wässriger Natronlauge zwischen 7,5 und 8,0 gehalten wird. Dann werden 500 ml Essigester zugegeben und das pH mit 2-n wässriger Salzsäure auf 1,5 eingestellt. Nach dem Abtrennen der organischen Phase wird die wässrige Phase einmal mit Essigester extrahiert. Die vereinigten organischen Phasen werden zweimal mit gesättigter wässriger Kochsalz-Lösung gewaschen, über Natriumsulfat getrocknet und auf ein Volumen von ca. 100 ml eingeengt. Der Rückstand wird von Ungelöstem abfiltriert ud das erhaltene orangegefärbte Filtrat mit 1000 ml Aether verdünnt. Die dabei amorph ausgefallene (7R) - 7 - [2 - (2 - furyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure wird abgenutscht und mit Aether sowie tiefsiedendem Petroläther gewaschen. Das erhaltene beige-farbene Produkt wird in 250 ml Essigester gelöst und von Ungelöstem abfiltriert. Das orangefärbte Filtrat wird mit 10 ml einer 2-n Lösung von 2-Aethylcapronsäure-Natriumsalz in Essigester versetzt, wobei das Natriumsalz der (7R) - 7 - [2 - (2 - Furyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure ausfällt. Dieses wird abgenutscht, mit Essigester und tiefsiedendem Petroläther gewaschen und 2 Tage im Vakuum bei 25°C getrocknet. Das erhaltene Produkt ist ein beigefarbenes Pulver (syn/anti-Gemisch 80:20). $[\alpha]_D^{20} = -108{,}6°$ (c = 0,5 in Wasser). $R_f$-Wert = 0,10 (DC auf Kieselgel-$F_{254}$-Fertigplatten im System Butanol/Eisessig/Wasser 4:1:1; Sichtbarmachung mit UV-Licht).

Beispiel 2

Herstellung des Natriumsalzes der (7R) - 7 - [2 - (2 - Furyl) - 2 - (methoxyimino)acetamido] - 3 - /[1 - amino - 1,2 - dihydro - 2 - oxo - 4 - pyrimidinyl)thio] - methyl/ - 3 - cephem - 4 - carbonsäure:

Diese Verbindung wird analog Beispiel 1 aus 3,4 g 2-Methoxyimino-2-furyl-essigsäure und 7,11 g (7R) - 7 - Amino - 3 - desacetoxy - 3 - [(1 - amino - 1,2 - dihydro - 2 - oxo - 4 - pyrimidinyl) - thio] - cephalosporansäure hergestellt. Das Produkt stellt ein beiges Pulver dar (syn/anti-Gemisch 70:30), $R_f$-Wert = 0,40 (DC auf Kieselgel-$F_{254}$—Fertigplatten im System Butanol/Eisessig/Wasser 4:1;1; Sichtbarmachung mit UV-Licht).

Beispiel 3

Herstellung des Natriumsalzes der (7R) - 7 - [2 - (Phenyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure:

Diese Verbindung wird analog Beispiel 1 aus 1,8 g 2-Methoxyimino-phenyl-essigsäure (syn/anti-Gemisch 90:10) und 3,73 g (7R) - 7 - Amino - 3 - desacetoxy - 3 - [(1,2,5,6 - tetrahydro - 2 - methyl - 5,6 - dioxo - as - triazin - 3 - yl) - thio] - cephalosporansäure hergestellt. Das Produkt ist ein beiges Pulver (syn/anti-Gemisch 90:10). $[\alpha]_D^{20} = -135°$ (c = 0,5 in Wasser). $R_f$-Wert = 0,17 (DC auf Kieselgel-$F_{254}$-Fertigplatten im System Butanol/Eisessig/Wasser 4:1:1, Sichtbarmachung mit UV-Licht).

Beispiel 4

Herstellung des Natriumsalzes der (7R) - 7 - [2 - (2 - Furyl) - 2 - (methoxyimino)acetamido] - 3 - /[(1,4,5,6 - tetrahydro - 4 - methyl - 5,6 - dioxo - as - triazin - 3 - yl)thio]methyl/ - 3 - cephem - 4 - carbonsäure:

Diese Verbindung wird analog Beispiel 1 aus 3,4 g 2-Methoxyimino-2-furyl-essigsäure (syn/anti-Gemisch 80:20) und 7,46 g (7R) - 7 - Amino - 3 - desacetoxy - 3 - [(1,4,5,6 - tetrahydro - 4 - methyl - 5,6 - dioxo - as - triazin - 3 - yl) - thio] - cephalosporansäure hergestellt. Das Produkt ist ein beiges Pulver (syn/anti-Gemisch 80:20). $[\alpha]_D^{20} = -44°$ (c = 0,5 in Wasser). $R_f$-Wert = 0,34 (DC auf Kieselgel-$F_{254}$-Fertigplatten im System Butanol/Eisessig/Wasser 4:1:1; Sichtbarmachung mit UV-Licht).

Beispiel 5

Herstellung des Natriumsalzes der (7R) - 7 - [2 - (Methoxyimino) - 2 - (2 - thienyl)acetamido] - 3 - /[1,2,5,6 - tetrahydro - 2 - methyl - 5,6 - dioxo - as - triazin - 3 - yl)thio]methyl/ - 3 - cephem - 4 - carbonsäure:

Diese Verbindung wird analog Beispiel 1 aus 1,85 g 2-Methoxyimino-2-thienyl-essigsäure (syn/anti-Gemisch ca. 70:30) und 3,71 g (7R) - 7 - Amino - 3 - desacetoxy - 3 - [(1,2,5,6 - tetrahydro - 2 - methyl - 5,6 - dioxo - as - triazin - 3 - yl)thio] - cephalosporansäure hergestellt. Das Produkt ist ein beiges Pulver (syn/anti-Gemisch ca. 70:30). $[\alpha]_D^{20} = -128{,}2°$ (c = 0.5 in Wasser). $R_f$-Wert s 0,21 (DC auf Kieselgel-$F_{254}$-Fertigplatten im System Butanol/Eisessig/Wasser 4:1:1, Sichtbarmachung mit UV-Licht).

Beispiel 6

Herstellung des Dinatriumsalzes der (7R) - 7 - [2 - (2 - Furyl) - 2 - (methoxy imino)acetamido] - 3 - /[(2,5 - dihydro - 2 - methyl - 5 - oxo - 6 - hydroxy - as - triazin - 3 - yl)thio]methyl/ - 3 - cephem - 4 - carbonsäure:

Diese Verbindung wird analog Beispiel 1 aus 10,12 g 2-Methoxyimino-2-furyl-essigsäure (syn-Isomer) und 18,7 g (7R) - 7 - Amino - 3 - desacetoxy - 3 - [(1,2,5,6 - tetrahydro - 2 - methyl - 5,6 - dioxo - as - triazin - 3 - yl)thio] - cephalosporansäure hergestellt. Bei der Salzbildung werden 20 ml (2 Aequiv.) einer 2-n Lösung von 2-Aethylcapronsäure-Natriumsalz in Essigester verwendet. Das Produkt is ein praktisch farbloses Pulver (syn-Isomer). $[\alpha]_D^{20} = -141{,}6°$ (c $\mp$ 0,5 in Wasser). $R_f$-Wert = 0,14 (DC auf Kieselgel-$F_{254}$-Fertigplatten im System Butanol/Eisessig/Wasser 4:1:1, Sichtbarmachung mit UV-Licht).

Beispiel 7

Herstellung des Natriumsalzes der (7R) - 3 - /[(1-Aethyl - 1,4,5,6 - tetrahydro - 5,6 - dioxo - as - triazin - 3 - yl)thio]methyl/ - 7 - [2 - (2 - furyl) - 2 - (methoxyimino)acetamido] - 3 - cephem - 4 - carbonsäure:

Diese Verbindung wird analog Beispiel 1 aus 1,69 g 2-Methoxyimino-2-furyl-essigsäure (syn-Isomer) und 3,85 g (7R) - 7 - Amino - 3 - desacetoxy - 3 - [(1 - äthyl - 1,4,5,6 - tetrahydro -

5,6 - dioxo - as - triazin - 3 - yl)thio] - cephalosporansäure hergestellt. Das Produkt ist ein beiges Pulver (syn/anti-Gemisch ca. 70:30). $[\alpha]_D^{20} = -47,2°$ (c = 0,5 in Wasser), $r_f$-Wert = 0,47 (DC auf Kieselgel-$F_{254}$-Fertigplatten im System Butanol/Eisessig/Wasser 4:1:1, Sichtbarmachung mit UV-Licht).

Beispiel 8

Herstellung des Natriumsalzes der (7R) - 7 - /2 - [(Carbamoyl - methoxy)imino] - 2 - (2 - furyl)acetamido/ - 3 - /[1,4,5,6 - tetrahydro - 4 - methyl - 5,6 - dioxo - as - triazin - 3 - yl)thio]methyl/ - 3 - cephem - 4 - carbonsäure:

9,76 g /$\alpha$ - [(Carbamoylmethoxy)imino]furfuryl/cephalosporin Natriumsalz (syn/anti-Gemisch ca. 70:30) werden zusammen mit 4,77 g 1,4,5,6-Tetrahydro-4-methyl-5,6-dioxo-3-mercapto-as-triazin in 200 ml Phosphatpuffer mit pH 6,4 suspendiert. Unter Sticksoffbegasung wird das pH mittels 1-n Natronlauge auf 6,4 gestellt, wobei eine dunkle Lösung entsteht. Diese Lösung wird 6 Stunden bei 55—60°C unter Stickstoffbegasung bei pH 6,4—6,5 gerührt, wobei das pH mit Hilfe eines Autotitrators unter Zugabe von 1-n Natronlauge konstant gehalten wird. Die Reaktionslösung wird auf 0—5°C abgekühlt und das pH mit 2-n Salzsäure auf 2 gestellt, wobei das Reaktionsprodukt als Säure ausfällt. Dieses wird abgenutscht, mit Eiswasser gewaschen und über Nacht im Vakuum bei 40°C getrocknet. Man erhält das Endprodukt in Form der rohen Säure. Zur Reinigung wird diese in 150 ml Methanol gelöst und die Lösung 2 Minuten mit Aktivkohle gekocht. Das Gemisch wird durch ein Faltenfilter filtriert und das orangefarbige Filtrat im Vakuum eingeengt. Das dabei ausgeschiedene Harz wird abgetrennt und verworfen. Die konzentrierte methanolische Lösung wird auf Aether gegossen. Die dabei ausgefallene Säure wird abgenutscht, mit Aether und tiefsiedendem Petroläther gewaschen. Man erhält das Endprodukt in Form der gereinigten Säure, die, zwecks Ueberführung in das Natriumsalz, in 100 ml Methanol gelöst wird und mit 5 ml einer 2-n Lösung von 2-Aethylcapronsäure-Natriumsalz in Essigester versetzt wird. Es wird von wenig Ungelöstem abfiltriert und das orangefarbige Filtrat im Vakuum bei 40°C eingeengt. Dieser konzentrierten Lösung wird Aethanol zugegeben, wobei das Natriumsalze ausfällt. Dieses wird abgenutscht, mit Aethanol und tiefsiedendem Petroläther gewaschen und über Nacht im Vakuum bei 40°C getrocknet. Man erhält das Natriumsalz der (7R) - 7 - /2 - [(Carbamoylmethoxy)imino] - 2 - (2 - furyl)acetamido/ - 3 - /[1,4,5,6 - tetrahydro - 4 - methyl - 5,6 - dioxo - as - triazin - 3 - yl)thio]methyl/ - 3 - cephem - 4 - carbonsäure als beiges Pulver (syn/anti-Gemisch ca. 70:30). $[\alpha]_D^{20} = -30,1°$ (c = 1 in Wasser), $R_f = 0,29$ (DC auf Kieselgel-$F_{254}$-Fertigplatten im System Butanol/Eisessig/Wasser 4:1:1, Sichtbarmachung mit UV-Licht).

Ersetzt man die Ausgangsverbindungen durch äquivalente Mengen /$\alpha$ - [(Methoxy)imino]furfuryl/cephalosporin und 1,2,5,6 - Tetrahydro - 2 - methyl - 5,6 - dioxo - 3 - mercapto - as - triazin, so erhält man unter sonst gleichen Bedingungen das Natriumsalz der (7R) - 7 - [2 - (2 - Furyl) - 2 - (methoxyimino)acetamido] - 3 - /[1,2,5,6 - tetrahydro - 2 - methyl - 5,6 - dioxo - as - triazin - 3 - yl)thio]methyl/ - 3 - cephem - 4 - carbonsäure. Das Produkt ist mit der gemäss Beispiel 1 erhaltenen Verbindung identisch.

Beispiel 9

Herstellung von Trockenampullen für die intramuskuläre Verabreichung;

Es wird in üblicher Weise ein Lyophilisat von 1 g des Natriumsalzes der (7R) - 7 - [2 - (2 - Furyl) - 2 - (methoxyimino)acetamido] - 3 - /[1,2,5,6 - tetrahydro - 2 - methyl - 5,6 - dioxo - as - triazin - 3 - yl)thio]methyl/ - 3 - cephem - carbonsäure hergestellt und in eine Ampulle abgefüllt. Vor der Verabreichung wird letzteres mit 2,5 ml einer 2%igen wässrigen Lidocainhydrochlorid-Lösung versetzt.

**Patentansprüche**

1. Cephalosporinderivate der allgemeinen Formel

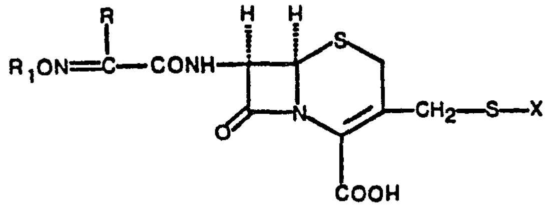

I

in der R Furyl, Thienyl oder ggfs. durch Halogen, Hydroxy, niederes Alkoxy oder niederes Alkyl substituiertes Phenyl, $R_1$ niederes Alkyl oder Aminocarbonylmethyl und X eine Gruppe der Formeln

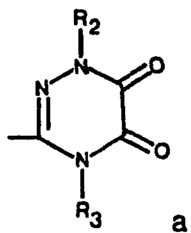 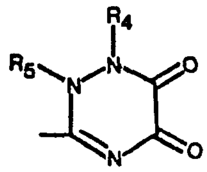 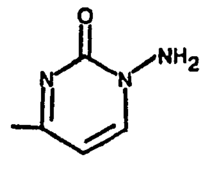

a b c

in denen einer der beiden Reste $R_2$ und $R_3$ bzw. $R_4$ und $R_5$ Wasserstoff und der andere niederes Alkyl, Carboxymethyl oder Sulfomethyl darstellt, bedeutet, sowie Salze dieser Verbindungen und Hydrate dieser Salze.

2. Derivate nach Anspruch 1, dadurch gekennzeichnet, dass R Furyl darstellt, sowie Salze dieser Verbindungen und Hydrate dieser Salze.

3. Derivate nach Anspruch 1 oder 2, dadurch gekennzeichnet, dass $R_1$ Methyl darstellt, sowie Salze dieser Verbindungen und Hydrate dieser Salze.

4. Derivate nach einem der Ansprüche 1—3, dadurch gekennzeichnet, dass X die Gruppe (c) oder eine der Gruppen (a) und (b), in denen einer der beiden Reste $R_2$ und $R_3$ bzw. $R_4$ und $R_5$ Wasserstoff und der andere Methyl bedeutet, darstellt, sowie Salze dieser Verbindungen und Hydrate dieser Salze.

5. Derivate nach Anspruch 4, dadurch gekennzeichnet, dass X eine 1,2,5,6-Tetrahydro-2-methyl-5,6-dioxo-as-triazin-3-yl-gruppe darstellt, sowie Salze dieser Verbindungen und Hydrate dieser Salze.

6. Derivate nach Anspruch 4, dadurch gekennzeichnet, dass X die 1,4,5,6-Tetrahydro-4-methyl-5,6-dioxo-as-triazin-3-yl-gruppe darstellt, sowie Salze dieser Verbindungen und Hydrate dieser Salze.

7. (7R) - 7 - [2 - (2 - Furyl) - 2 - (methoxyimino)acetamido] - 3 - /[(1,2,5,6 - tetrahydro - 2 - methyl - 5,6 - dioxo - as - triazin - 3 - yl)thio] - methyl/ - 3 - cephem - 4 - carbonsäure sowie Salze dieser Verbindung und Hydrate dieser Salze.

8. Verfahren zur Herstellung von Cephalosporinderivaten der allgemeinen Formel

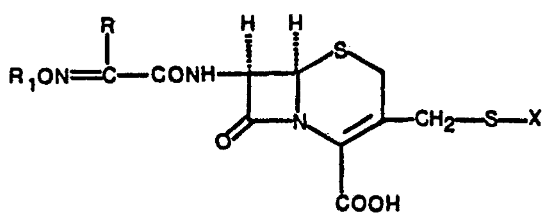 I

in der R Furyl, Thienyl oder ggfs. durch Halogen, Hydroxy, niederes Alkoxy oder niederes Alkyl substituiertes Phenyl, $R_1$ niederes Alkyl oder Aminocarbonylmethyl und X eine Gruppe der Formeln

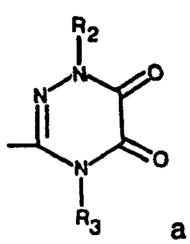 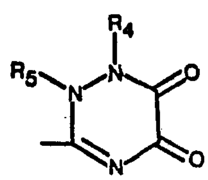 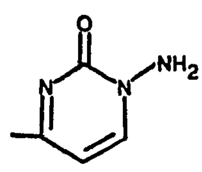

a b c

in denen einer der beiden Reste $R_2$ und $R_3$ bzw. $R_4$ und $R_5$ Wasserstoff und der andere niederes Alkyl, Carboxymethyl oder Sulfomethyl darstellt, bedeutet, sowie von Salzen dieser Verbindungen und von Hydraten dieser Salze, dadurch gekennzeichnet, dass man

a) eine Verbindung der allgemeinen Formel

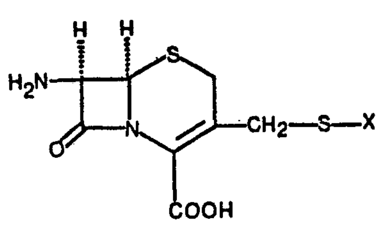 II

in der X die in Formel I gegebene Bedeutung hat und die Carboxygruppe in geschützter Form vorliegen kann, mit einer Säure der allgemeinen Formel

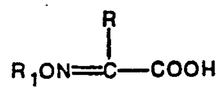

III

in der R und $R_1$ die in Formel I gegebene Bedeutung haben, oder mit einem reaktionsfähigen funktionellen Derivat dieser Säure umsetzt und die gegebenenfalls vorhandene Schutzgruppe abspaltet, oder dass mann

b) eine Verbindung der allgemeinen Formel

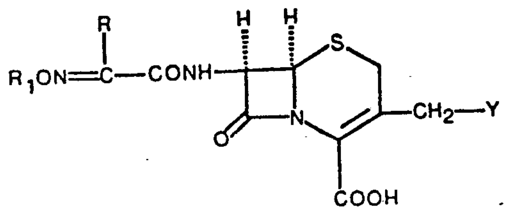

IV

in der R und $R_1$ die in Formel I gegebene Bedeutung haben, Y eine austretende Gruppe darstellt und die Carboxygruppe in geschützter Form vorliegen kann, mit einem Thiol der allgemeinen Formel

HS—X V

in der X die in Formel I gegebene Bedeutung hat, umsetzt und die gegebenenfalls vorhandene Schutzgruppe abspaltet, wonach man das Reaktionsprodukt ggfs. in ein Salz bzw. ein Hydrat dieses Salzes überführt.

9. Verfahren nach Anspruch 8, dadurch gekennzeichnet, dass man von einer Ausgangsverbindung der Formel II und einer Säure der Formel III oder einem reaktionsfähigen funktionellen Derivat einer solchen Säure ausgeht.

10. Verfahren nach Anspruch 9, dadurch gekennzeichnet, dass man das Chlorid einer Säure der Formel III mit einer Ausgangsverbindung der Formel II in wässrigem Alkali umsetzt.

11. Verfahren nach einem der Ansprüche 8—10 zur Herstellung von Verbindungen der Formel I, worin R Furyl darstellt, sowie von deren Salzen bzw. von Hydraten dieser Salze, dadurch gekennzeichnet, dass man entsprechend substituierte Ausgangsverbindungen einsetzt.

12. Verfahren nach einem der Ansprüche 8—11 zur Herstellung von Verbindungen der Formel I, worin $R_1$ Methyl darstellt, sowie von deren Salzen bzw. von Hydraten dieser Salze, dadurch gekennzeichnet, dass man entsprechend substituierte Ausgangsverbindungen einsetzt.

13. Verfahren nach einem der Ansprüche 8—12 zur Herstellung von Verbindungen der Formel I, worin X die Gruppe (c) oder eine der Gruppen (a) und (b), in denen einer der beiden Reste $R_2$ und $R_3$ bzw. $R_4$ und $R_5$ Wasserstoff und der andere Methyl bedeutet, darstellt, sowie von deren Salzen bzw. von Hydraten dieser Salze, dadurch gekennzeichnet, dass man entsprechend subtituierte Ausgangsverbindungen einsetzt.

14. Verfahren nach Anspruch 13 zur Herstellung von Verbindungen der Formel I, worin X eine 1,2,5,6 - Tetrahydro - 2 - methyl - 5,6 - dioxo - as - triazin - 3 - yl - gruppe darstellt, sowie von deren Salzen bzw. von Hydraten dieser Salze, dadurch gekennzeichnet, dass man entsprechend substituierte Ausgangsverbindungen einsetzt.

15. Verfahren nach Anspruch 13 zur Herstellung von Verbindungen der Formel I, worin X die 1,4,5,6 - Tetrahydro - 4 - methyl - 5,6 - dioxo - as - triazin - 3 yl - gruppe darstellt, sowie von deren Salzen bzw. von Hydraten dieser Salze, dadurch gekennzeichnet, dass man entsprechend substituierte Ausgangsverbindungen einsetzt.

16. Verfahren nach Anspruch 14 zur Herstellung von (7R) - 7 - [2 - (2 - Furyl) - 2 - (methoxyimino)acetamido] - 3 - /[(1,2,5,6 - tetrahydro - 2 - methyl - 5,6 - dioxo - as - triazin - 3 - yl)thio]methyl/ - 3 - cephem - 4 - carbonsäure sowie von deren Salzen bzw. von Hydraten dieser Salze, dadurch gekennzeichnet, dass man entsprechend substituierte Ausgangsverbindungen einsetzt.

17. Pharmazeutische Präparate, gekennzeichnet durch einen Gehalt an einer Verbindung der allgemeinen Formel

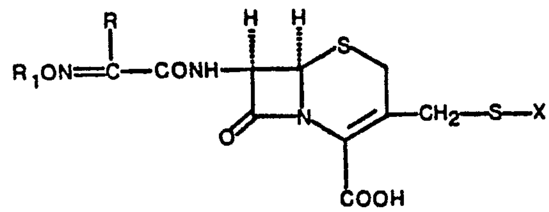

I

in der R Furyl, Thienyl oder ggfs. durch Halogen, Hydroxy, niederes Alkoxy oder niederes Alkyl substituiertes Phenyl, $R_1$ neideres Alkyl oder Aminocarbonylmethyl und X eine Gruppe der Formeln

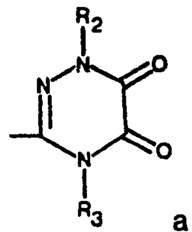

a

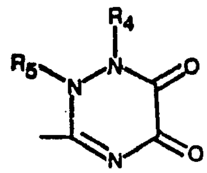

b

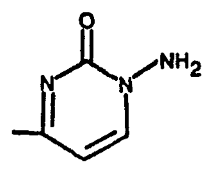

c

in denen einer der beiden Reste $R_2$ und $R_3$ bzw. $R_4$ und $R_5$ Wasserstoff und der andere niederes Alkyl, Carboxymethyl oder Sulfomethyl darstellt, bedeutet, oder an einem pharmazeutisch verträglichen Salz davon bzw. an einem Hydrat eines solchen Salzes.

**Revendications**

1. Les dérivés de céphalosporine de formule générale

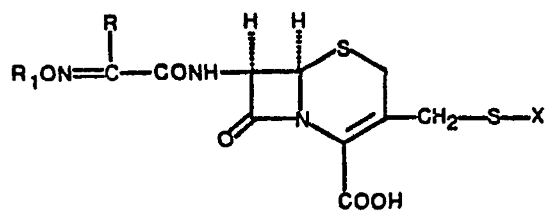

I

où R représente un furyle, un thiényle ou un phényle éventuellement substitué par un halogène, un hydroxy, un alcoxy inférieur ou un alcoyle inférieur, $R_1$ représente un alcoyle inférieur ou un aminocarbonylméthyle et X un groupe de formules

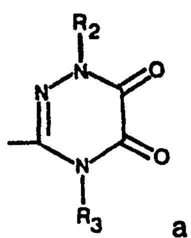

a

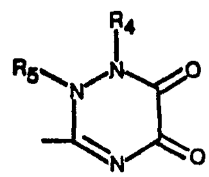

b

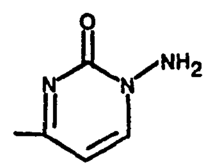

c

où l'un des deux radicaux $R_2$ et $R_3$ ou $R_4$ et $R_5$ représente un hydrogène et l'autre un alcoyle inférieur, un carboxyméthyle ou un sulfométhyle, ainsi que les sels de ces composés et les hydrates de ces sels.

2. Les dérivés selon la revendication 1, caractérisés en ce que R représente un furyle, ainsi que les sels de ces composés et les hydrates de ces sels.

3. Les dérivés selon la revendication 1 ou 2, caractérisés en ce que $R_1$ représente le méthyle, ainsi que les sels de ces composés et les hydrates de ces sels.

4. Les dérivés selon l'une des revendications 1—3, caractérisés en ce que X représente le groupe (c) ou l'un des groupes (a) et (b), dans lesquels l'un des radicaux $R_2$ et $R_3$ ou $R_4$ et $R_5$ représente un hydrogène et l'autre le méthyle, ainsi que les sels de ces composés et les hydrates de ces sels.

5. Les dérivés selon la revendication 4, caractérisés en ce que X représente le groupe 1,2,5,6 - tétrahydro - 2 - méthyl - 5,6 - dioxo - as - triazin - 3 - yle, ainsi que les sels de ces composés et les hydrates de ces sels.

6. Les dérivés selon la revendication 4, caractérisés en ce que X représente le groupe 1,4,5,6 -

tétrahydro - 4 - méthyl - 5,6 - dioxo - as - triazin - 3 - yle, ainsi que les sels de ces composés et les hydrates de ces sels.

7. L'acide (7R) - 7 - [2 - (2 - furyl) - 2 - (méthoxyimino) - acétamido] - 3 - /[1,2,5,6 - tétrahydro - 2 - méthyl - 5,6 - dioxo - as - triazin - 3 - yl)thio] - methyl/ - 3 - céphem - 4 - carboxylique, ainsi que les sels de ce composé et les hydrates de ces sels.

8. Procédé pour la préparation de dérivés de céphalosporine de formule générale

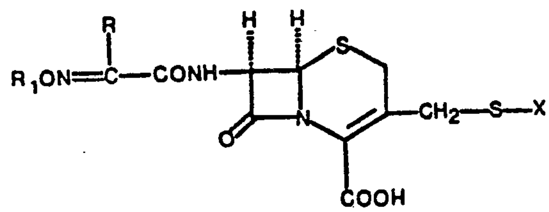

I

où R représente un furyle, un thiényle ou un phényle éventuellement substitué par un halogène, un hydroxy, un alcoxy inférieur ou un alcoyle inférieur, $R_1$ représente un alcoyle inférieur ou un aminocarbonyl-méthyle et X un groupe de formules

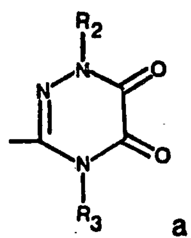

a

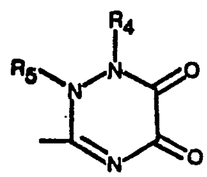

b

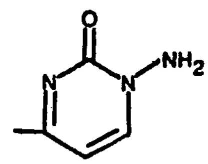

c

où l'un des deux radicaux $R_2$ et $R_3$ ou $R_4$ et $R_5$ représente un hydrogène et l'autre un alcoyle inférieur, un carboxyméthyle ou un sulfométhyle, ainsi que les sels de ces composés et les hydrates de ces sels, caractérisé en ce que

a) on fait réagir un composé de formule générale

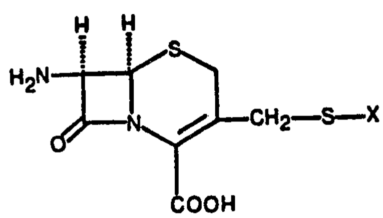

II

où X a la signification donnée dans la formule I et où le groupe carboxy peut se présenter sous forme protégée, avec un acide de formule générale

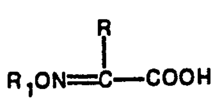

III

où R et $R_1$ ont la signification donnée dans la formule I, ou avec un dérivé fonctionnel réactif de cet acide et on sépare le groupe protecteur éventuellement présent, ou

b) on fait réagir un composé de formule générale

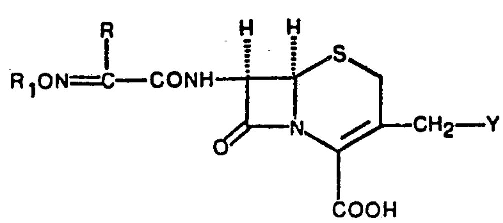

IV

où R et $R_1$ ont la signification donnée dans la formule I, Y représente un groupe détachable et où le groupe carboxy peut se présenter sous forme protégée, avec un thiol de formule générale

HS—X V

où X a la signification donnée dans la formule I , et on sépare le groupe protecteur éventuellement présent, après quoi on transforme, le cas échéant, le produit réactionnel en un sel ou en un hydrate de ce sel.

9. Un procédé selon la revendication 8, caractérisé en ce que l'on part d'un produit de départ de formule II et d'un acide de formule III ou d'un dérivé fonctionnel réactif d'un tel acide.

10. Un procédé selon la revendication 9, caractérisé en ce que l'on fait réagir le chlorure d'un acide de formule III avec un produit de départ de formule II dans un alcali aqueux.

11. Un procédé selon l'une des revendications 8—10 de préparation de composés de formule I, où R représente un furyle, ainsi que de leurs sels ou des hydrates de ces sels, caractérisé en ce que l'on utilise des produits de départ substitués correspondants.

12. Un procédé selon l'une des revendications 8—11 de préparation de composés de formule I, où $R_1$ représente un méthyle, ainsi que de leurs sels ou des hydrates de ces sels, caractérisé en ce qu'on utilise des produits de départ substitués correspondants.

13. Un procédé selon l'une des revendications 8—12 de préparation de composés de formule I, où X représente le groupe (c) ou un des groupes (a) et (b), dans lesquels l'un des deux radicaux $R_2$ et $R_3$ ou $R_4$ et $R_5$ représente un hydrogène ou l'autre un méthyle, ainsi que de leurs sels ou des hydrates de ces sels, caractérisé en ce que l'on utilise des composés de départ substitués correspondants.

14. Un procédé selon la revendication 13 de préparation de composés de formule I, où X représente un groupe 1,2,5,6-tétrahydro-2-méthyl-5,6-dioxo-as-triazin-3-yle, ainsi que de leurs sels ou des hydrates de ces sels, caractérisé en ce que l'on utilise des produits de départ substitués correspondants.

15. Un procédé selon la revendication 13 de préparation de composés de formule I, où X représente le groupe 1,4,5,6-tétrahydro-4-méthyl-5,6-dioxo-as-triazin-3-yle, ainsi que de leurs sels ou des hydrates de ces sels, caractérisé en ce que l'on utilise des produits de départ substitués correspondants.

16. Un procédé selon la revendication 14 de préparation de l'acide (7R) - 7 - [2 - (2 - furyl) - 2 - (méthoxyimino) - acétamido] - 3 - /[(1,2,5,6 - tétrahydro - 2 - méthyl - 5,6 - dioxo - as - triazin - 3 - yl)thio] - méthyl/ - 3 - céphem - 4 - carboxylique, ainsi que de ses sels ou des hydrates de ces sels, caractérisé en ce que l'on utilise des composés de départ substitués correspondants.

17. Préparations pharmaceutiques, caractérisées en ce qu'elles contiennent un composé de formule générale

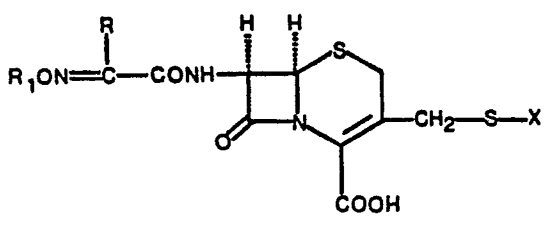

I

où R représente un furyle, un thiényle ou un phényle éventuellement substitué par un halogène, un hydroxy, un alcoxy inférieur ou un alcoyle inférieur, $R_1$ représente un alcoyle inférieur ou un amino-carbonylméthyle et X un groupe de formules

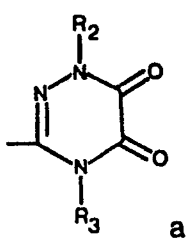

a

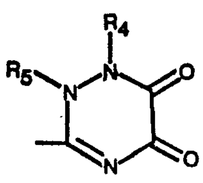

b

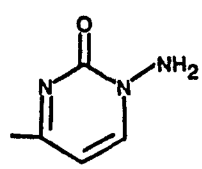

c

où l'un des deux radicaux $R_2$ et $R_3$ ou $R_4$ et $R_5$ représente un hydrogène et l'autre un alcoyle inférieur, un carboxyméthyle ou un sulfométhyle, ou un sel pharmaceutiquement compatible de ce composé ou un hydrate d'un tel sel.

## Claims

1. Cephalosporin derivatives of the general formula

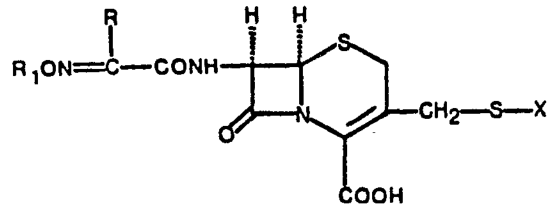

I

wherein R represents furyl, thienyl or phenyl optionally substituted by halogen, hydroxy, lower alkoxy or lower alkyl, $R_1$ represents lower alkyl or aminocarbonylmethyl and X represents a group of the formulae

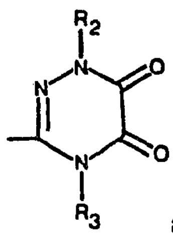

a

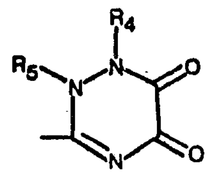

b

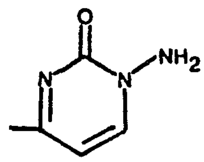

c

in which one of the two symbols $R_2$ and $R_3$ or $R_4$ and $R_5$ represents hydrogen and the other represents lower alkyl, carboxymethyl or sulphomethyl, as well as salts of said compounds and hydrates of said salts.

2. Derivatives according to claim 1, characterized in that R represents furyl, as well as salts of said compounds and hydrates of said salts.

3. Derivatives according to claim 1 or 2, characterized in that $R_1$ represents methyl, as well as salts of said compounds and hydrates of said salts.

4. Derivatives according to one of claims 1 to 3, characterized in that X represents the group (c) or one of groups (a) and (b), in which one of the two symbols $R_2$ and $R_3$ or $R_4$ and $R_5$ represents hydrogen and the other represents methyl, as well as salts of said compounds and hydrates of said salts.

5. Derivatives according to claim 4, characterized in that X represents a 1,2,5,6-tetrahydro-2-methyl-5,6-dioxo-as-triazin-3-yl group, as well as salts of said compounds and hydrates of said salts.

6. Derivatives according to claim 4, characterized in that X represents the 1,4,5,6-tetrahydro-4-methyl-5,6-dioxo-as-triazin-3-yl group, as well as salts of said compounds and hydrates of said salts.

7. (7R) - 7 - [2 - (2 - Furyl) - 2 - (methoxyimino)acetamido] - 3 - {[(1,2,5,6 - tetrahydro - 2 - methyl - 5,6 - dioxo - as - triazin - 3 - yl)thio]methyl} - 3 - cephem - 4 - carboxylic acid, as well as salts of said compound and hydrates of said salts.

8. Process for the manufacture of cephalosporin derivatives of the general formula

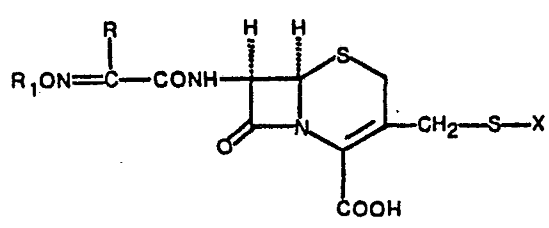

I

wherein R represents furyl, thienyl or phenyl optionally substituted by halogen, hydroxy, lower alkoxy or lower alkyl, $R_1$ represents lower alkyl or aminocarbonylmethyl and X represents a group of the formulae

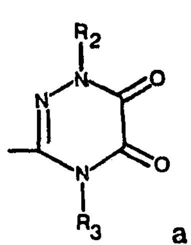

a

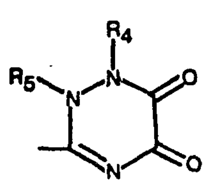

b

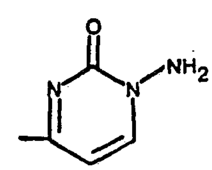

c

in which one of the two symbols $R_2$ and $R_3$ or $R_4$ and $R_5$ represents hydrogen and the other represents lower alkyl, carboxymethyl or sulphomethyl, as well as salts of said compounds and hydrates of said salts, which process comprises

(a) reacting a compound of the general formula

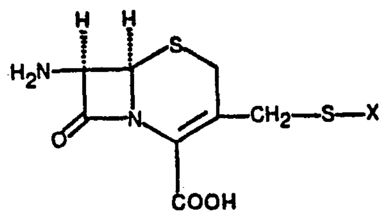 II

wherein X has the significance given in formula I and the carboxy group can be present in protected form, with an acid of the general formula

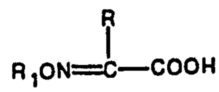 III

wherein R and $R_1$ have the significance given in formula I, or with a reactive functional derivative of this acid, and cleaving off the protecting group that may be present, or

(b) reacting a compound of the formula

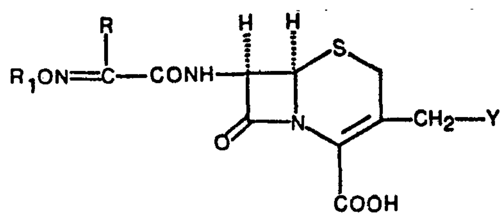 IV

wherein R and $R_1$ have the significance given in formula I, Y represents a leaving group and the carboxy group can be present in protected form, with a thiol of the general formula

HS—X V

wherein X has the significance given in formula I, and cleaving off the protecting group that may be present and if desired, converting the reaction product into a salt or a hydrate of such salt.

9. A process according to claim 8, characterized in that a starting compound of formula II is reacted with an acid of formula III or a reactive functional derivative of such acid.

10. A process according to claim 9, characterized in that the chloride of an acid of formula III is reacted with a starting compound of formula II in aqueous alkali.

11. A process according to one of claims 8 to 10 for the manufacture of compounds of formula I, in which R represents furyl, and of salts thereof or of hydrates of such salts, characterized in that appropriately substituted starting compounds are used.

12. A process according to one of claims 8 to 11 for the manufacture of compounds of formula I, in which $R_1$ represents methyl, and of salts thereof or of hydrates of such salts, characterized in that appropriately substituted starting compounds are used.

13. A process according to one of claims 8 to 12 for the manufacture of compounds of formula I, in which X represents the group (c) or one of groups (a) and (b), in which one of the two symbols $R_2$ and $R_3$ or $R_4$ and $R_5$ represents hydrogen and the other represents methyl, and of salts thereof or of hydrates of such salts, characterized in that appropriately substituted starting compounds are used.

14. A process according to claim 13 for the manufacture of compounds of formula I, in which X represents a 1,2,5,6-tetrahydro-2-methyl-5,6-dioxo-as-triazin-3-yl group, and of salts thereof or of hydrates of such salts, characterized in that appropriately substituted starting compounds are used.

15. A process according to claim 13 for the manufacture of compounds of formula I, in which X represents the 1,4,5,6-tetrahydro-4-methyl-5,6-dioxo-as-triazin-3-yl group, and of salts thereof or of hydrates of such salts, characterized in that appropriately substituted starting compounds are used.

16. A process according to claim 14 for the manufacture of (7R) - 7 - [2 - (2 - furyl) - 2 - (methoxyimino)acetamido] - 3 - {[(1,2,5,6-tetrahydro - 2 - methyl - 5,6 - dioxo - as - triazin - 3 - yl)thio]methyl} - 3 - cephem - 4 - carboxylic acid, and of salts thereof or of hydrates of such

salts, characterized in that appropriately substituted starting compounds are used.

17. Pharmaceutical compositions, characterized in that they contain a derivative of the general formula

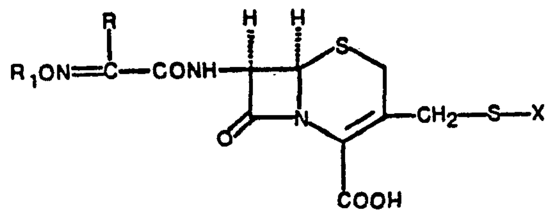

I

wherein R represents furyl, thienyl or phenyl optionally substituted by halogen, hydroxy, lower alkoxy or lower alkyl, $R_1$ represents a lower alkyl or aminocarbonylmethyl and X represents a group of the formulae

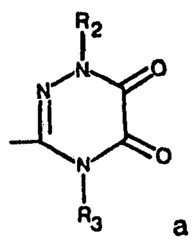

a

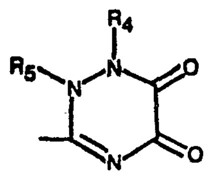

b

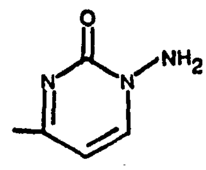

c

in which one of the two symbols $R_2$ and $R_3$ or $R_4$ and $R_5$ represents hydrogen and the other represents lower alkyl, carboxymethyl or sulphomethyl, or a pharmaceutically compatible salt thereof or a hydrate of such salt.